# EUROPEAN PATENT SPECIFICATION

(11) **EP 1 849 746 B1**
(45) Date of publication and mention of the grant of the patent: **13.06.2012**
(21) Application number: 07007875.3
(22) Date of filing: 18.04.2007
(51) Int. Cl.: B67C 7/00, A61L 2/08, A61L 2/20

(54) **Apparatus and method for sterilization of vessels**
Vorrichtung und Verfahren zur Sterilisation von Gefäßen
Appareil et procédé pour la stérilisation de récipients

(30) Priority: 26.04.2006 JP 2006122499
(43) Date of publication of application: 31.10.2007
(73) Proprietor: SHIBUYA KOGYO CO., LTD, Kanazawa-shi, Ishikawa-ken (JP)
(72) Inventor: Naka, Toshiaki, Kanazawa-shi Ishikawa-ken (JP); Nishino, Yukinobu, Kanazawa-shi Ishikawa-ken (JP); Nishi, Tokuo, Kanazawa-shi Ishikawa-ken (JP)
(74) Representative: HOFFMANN EITLE

(56) References cited:
- WO-A-2004/110869
- FR-A- 2 865 135
- JP-A- 58 216 527

## Description

### Field of the Invention

The present invention relates to a vessel sterilizer and a method of sterilization of vessels by irradiation of vessels with an electron beam.

### Background of the Invention

Techniques for sterilization of articles such as vessels, films or the like by irradiation with an electron beam is known in the art (see Japanese Laid-Open Patent Application No. 2006-61558 and Patent Granted Publication No. 1-17935 (17935/1989), for example). "Electron beam sterilizer" according to the invention disclosed in the cited Laid-Open Patent Application No. 2006-61558 includes a scan horn irradiating an electron beam and which is installed within a clean room filled with pasteurized air to irradiate PET bottles being conveyed with the electron beam for purpose of sterilization.

"Electron beam sterilizer for packaging materials" according to the invention disclosed in the cited Patent Granted Publication No. 1-17935 includes an electron beam irradiator disposed within an electron beam sterilization chamber for sterilization of packaging materials by irradiation, and a duct disposed in the sterilization chamber for feeding N2 gas to maintain a positive pressure in the interior of the sterilization chamber relative to the surrounding atmosphere. The sterilization chamber is cleaned, heated and sprayed with H2O2 through a pre-sterilization line, then dried to be aseptic, and pasteurized N2 is introduced into the room.

With a sterilizer which utilizes the electron beam, the germicidal power is effective only in an area which is irradiated with the electron beam, but is absent elsewhere. Accordingly, if the interior of the chamber is maintained aseptic, there remains a likelihood that germs which attached to a vessel conveyed into the chamber before the sterilization takes place may be re-attached to the sterilized vessel if they remain floating in the chamber. In particular, in an arrangement where the sterilization takes place while the vessels are moving, there occurs an air flow around the vessels to increase the risk of floating germs, resulting in an increase in the likelihood that they are re-attached to the sterilized vessels. This illustrates an instance that the electron beam sterilization may be prevented for germs which are introduced by being attached to vessels which are to be sterilized, leaving a problem that the sterilizer exhibits a poor reliability.

### Summary of the Invention

According to a first aspect of the present invention, there is provided a vessel sterilizer as set forth in Claim 1 below.

According to a second aspect of the present invention, there is provided a method of sterilizing vessels as set forth in Claim 5 below.

Accordingly, it is an advantage achievable with embodiments of the present invention to provide a vessel sterilizer of a high reliability which avoids the likelihood of germs attached to vessels introduced into the sterilization chamber re-attaching, if they remain floating in the chamber, to vessels which are sterilized by the irradiation of the electron beam, by injecting a germicidal gas into the chamber.

### Brief Description of the Drawings

To enable a better understanding of the invention, and to show how the same may be carried into effect, reference will now be made, by way of example, to the following drawings, in which:
Fig. 1 is a plan view showing an arrangement of an entire sterilizing and filling line which is provided with a vessel sterilizer according to one embodiment of the present invention;
Fig. 2 is a perspective view showing vessel holder means which is provided in a vessel inversion conveyor of the vessel sterilizer of Fig. 1;
Fig. 3 is a cross section taken along line III-III shown in Fig. 1;
Fig. 4 is a cross section taken along line IV-IV shown in Fig. 1;
Fig. 5 is a cross section taken along line V-V shown in Fig. 1;
Fig. 6 is a view showing an inactive gas injection nozzle mounted on a second rotary wheel;
Fig. 7 is a view showing an aseptic air injection nozzle mounted on an intermediate wheel; and
Fig. 8 is a plan view showing an arrangement of an entire sterilizing and filling line which is provided with a vessel sterilizer according to a second embodiment.

### Detailed description

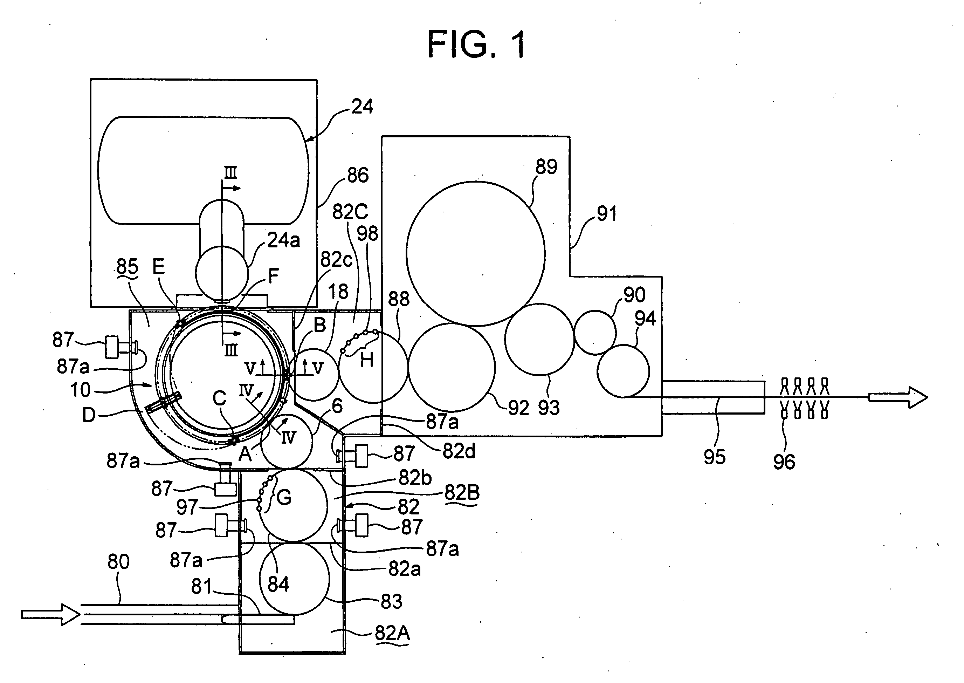

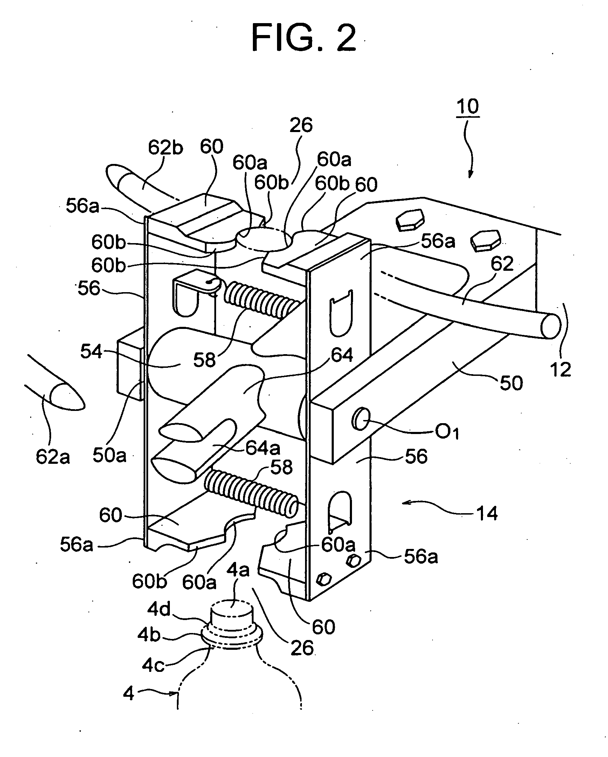

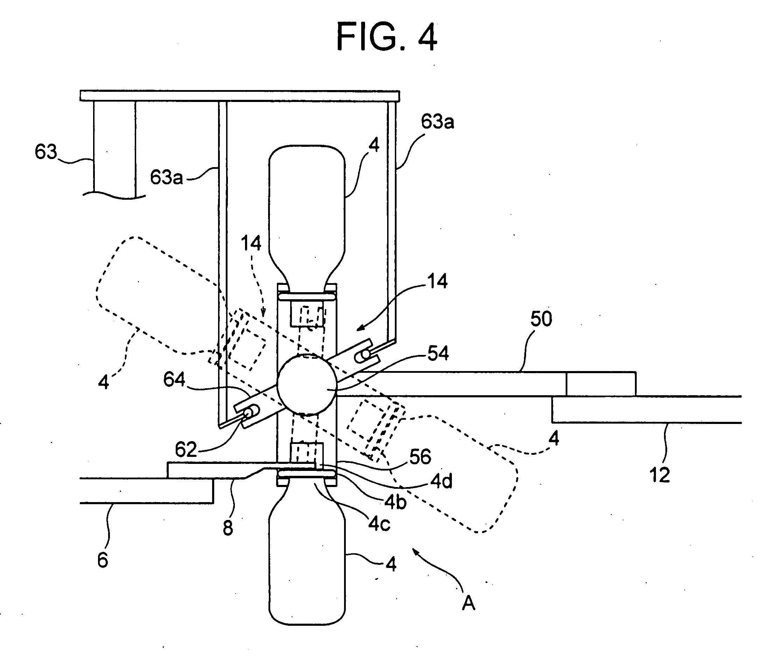

Several embodiments of the present invention shown in the drawings will now be described. Vessels 4 which are sterilized with the vessel sterilizer according to this embodiment represent PET bottles (to be described later with reference to Figs. 2-5), are continuously conveyed on an air conveyor 80, and are separated into a given interval by an in-feed screw 81 to be introduced into an aseptic chamber 82. A plurality of rotary wheels 83, 84 which are provided with vessel holder means (not shown) are disposed in an upstream portion of the aseptic chamber 82, and the vessels 4 which are introduced into the aseptic chamber 82 are successively handed to the rotary wheels 83, 84, and then handed to a gripper 8 (see Fig. 4) of a supply gripper wheel 6 to be conveyed rotatively, and subsequently are supplied to a vessel inversion conveyor 10 (vessel conveying means as recited in Claim 1) which inverts the vessels 4 while conveying them rotatively. A supply position where the vessel is fed from the supply gripper wheel 6 to the vessel inversion conveyor 10 is indicated by a character A in Fig. 1.

The aseptic chamber 82 is surrounded by a lead wall surface in order to avoid an exposure to the electron beam which is irradiated from an electron beam irradiator which will be described later. In the aseptic chamber 82 which is made from lead, a partition wall 82a partitions between the first rotary wheel 83 which is located toward the inlet where the vessel 4 is initially supplied and the second rotary wheel 84, and a partition wall 82b partitions between the second rotary wheel 84 and the supply gripper wheel 6 except for regions where the vessels 4 are handed over. In addition, a partition wall 82c partitions between the vessel inversion conveyor 10 and a discharge gripper wheel 18 which is located downstream to discharge vessels 4 from the vessel inversion conveyor 10 except for a region where the vessels 4 are handed over. In this manner, the supply gripper wheel 6 and the vessel inversion conveyor 10 are disposed within a chamber (electron beam irradiation chamber 85) which is partitioned by the upstream partition wall 82b and the downstream partition wall 82c.

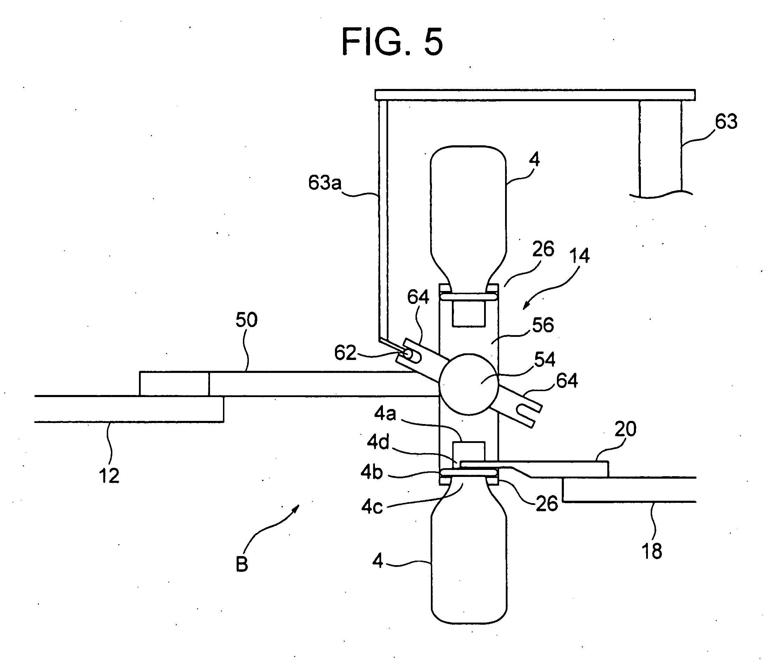

The vessel inversion conveyor 10 includes a revolving body 12 (see Fig. 2), and a plurality of vessel holder means 14 are mounted around the outer periphery of the revolving body at an equal circumferential interval. The vessels 4 which are fed from the supply gripper wheel 6 are carried by the vessel holder means 14, and are conveyed rotatively by rotatively shifting the vessel holder means 14 as the revolving body 12 rotates. Inversion means (not shown) is disposed along the conveying path of the vessels 4 for inverting the vessel holder means 14 which carries the vessels 4, whereby the vessels 4 which are supplied in an erect condition (the vessel 4 has its mouth 4a directed upward) is inverted by the inversion means to its inverted condition. Subsequently, the vessels 4 are rotatively conveyed in their inverted condition, and are again inverted by the inversion means to their erect condition to be handed to a gripper 20 (see Fig. 5) of a discharge gripper wheel 18 to be discharged. In this embodiment, at the vessel supply position (position A shown), the vessels 4 are handed over from the gripper 8 of the supply gripper wheel 6 to the vessel holder means 14 of the vessel inversion conveyor 10, inverted twice down and up during the time the revolving body 12 rotates through substantially two revolutions, and handed over to the gripper 20 of the discharge gripper wheel 18 at the vessel discharge position (position B shown).

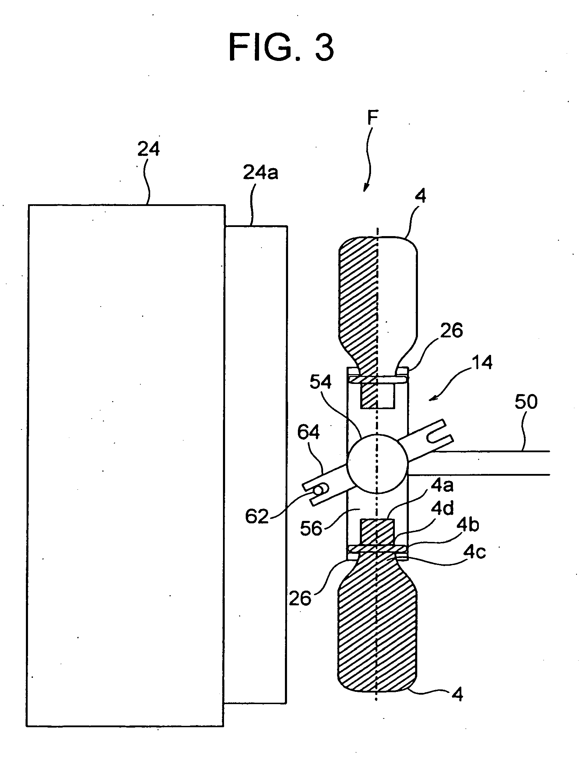

An electron beam irradiator 24 is disposed at a location upstream of the vessel discharge position B along the outer periphery of the vessel inversion conveyor 10. The electron beam irradiator 24 is contained within a chamber 86 which is separate from the aseptic chamber 82 which is made from lead, and includes an electron beam irradiation unit 24a (see Figs. 1 and 3) which is directed toward the center of the vessel inversion conveyor 10 which is disposed within the aseptic chamber 82. The arrangement of the electron beam irradiator 24 is well known in the art, and therefore will not be shown, and a detailed description therefor is omitted. However, briefly, it includes the irradiation unit 24a where a filament is heated in vacuum to generate thermions, which are accelerated under a high tension to form a rapid electron beam, which is taken out into the atmosphere through a window foil which is mounted on an irradiation window and which may be formed by titanium (Ti) to impinge upon an article to be treated (which is the vessel 4 in the present embodiment) to perform a desired treatment such as sterilization. As will be described later, the vessel holder means 14 includes a pair of upper and lower vessel holders 26, 26, and the irradiation unit 24a has a sufficient length to irradiate a pair of upper and lower vessels 4, 4 which are maintained in their upright condition by being carried by the pair of vessel holders 26, 26 simultaneously with the electron beam.

A plurality of H₂O₂ gas injector (hydrogen peroxide gas injectors) 87 are mounted on the wall surface of the electron beam irradiation chamber 85 of the aseptic chamber 82 which is made from lead for gasifying hydrogen peroxide solution to be injected from an injection nozzle 87a which is directed into the chamber 85. In the present embodiment, H₂O₂ gas injectors 87 are disposed at three locations on the wall surface of the electron beam irradiation chamber 85 to insure that the entire electron beam irradiation chamber 85 is uniformly filled with the germicidal H₂O₂ gas during the operation.

H₂O₂ gas injectors 87 are also mounted on the wall surface of the space in which the upstream, second rotary wheel 84 of the supply gripper wheel 6 is disposed, namely, the space which is partitioned between the upstream partition wall 82a and the downstream partition wall 82b for injecting gasified H₂O₂ from the injection nozzle 87a to provide a germicidal atmosphere in the space. Providing a germicidal atmosphere in the space which is located upstream of the electron beam irradiation chamber 85 where the sterilization by way of the electron beam takes place is effective to remove impurities such as germs previously.

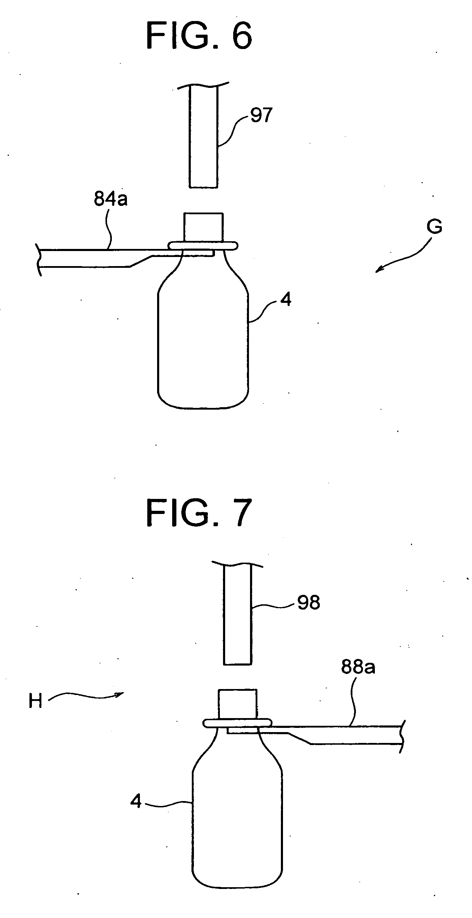

Inactive gas injection nozzles 97 (see Fig. 6) are disposed along part (a region indicated by character G in Fig. 1) of a conveying part of the second rotary wheel 84. An inactive gas such as nitrogen gas from the inactive gas injection nozzles 97 are blown into the vessels 4 which are handed from the first rotary wheel 83 to the second rotary wheel 84 during the time they are conveyed through the region G by being carried by the vessel holder means 84a on the second rotary wheel 84. When the inactive gas is blown in this manner, the amount of residue air which is present in the vessel 4 is reduced. The electron beam reacts with oxygen to generate ozone, which smells and is undesirable, and accordingly, the amount of ozone which may be generated by the irradiation of the electron beam is reduced. It is to be noted that the inactive gas injection nozzles 97 may be mounted on the supply gripper wheel 6 which is located downstream of the second rotary wheel 84.

After being sterilized by the electron beam irradiated from the electron beam irradiator 24 within the electron beam irradiation chamber 85, the vessels 4 are then handed over from the discharge gripper wheel 18 to an intermediate rotary wheel 88 to be discharged from the aseptic chamber 82 which is made from lead, the vessels being conveyed into a separate chamber 91 in which a filler 89 and a capper 90 are installed. The intermediate rotary wheel 88 is disposed so as to extend through a partition wall 82d which partitions between the aseptic chamber 82 located toward the electron beam sterilizer 24 and the chamber 91 in which the filler 89 and the like are installed. An aseptic air injection nozzle 98 (see Fig. 7) is disposed along part (region indicated by character H in Fig. 1) of a conveying path of the intermediate rotary wheel 88. The vessels 4 which are handed over from the discharge gripper wheel 18 to the intermediate rotary wheel 88 are carried by vessel holder means 88a on the intermediate rotary wheel 88, and the aseptic air is blown into the vessels from the aseptic air injection nozzle 98 during the time they are conveyed through the region H. If ozone which is generated in the electron beam irradiation chamber 85 is attached to the vessels 4, it can be blown off by the injection of the aseptic air. While the aseptic air injection nozzle 98 and the inactive gas injection nozzles 97 are mounted at fixed positions in the embodiment, an arrangement can be made such that these nozzles 97, 98 move in tracking relationship with the vessels 4 which are rotatively conveyed.

The vessels 4 which are rotatively conveyed by the intermediate rotary wheel 88 are handed to a supply wheel 92 to be fed to the filler 89. Subsequently, a liquid is filled into the vessels 4 during the time they are rotatively conveyed by the filler 89, and the vessels 4 are fed through a discharge wheel 93 from the filler 89 to a capper 90 where a capping operation takes place. Upon completion of the filling and the capping operation, the vessels 4 are handed through a delivery wheel 94 located at the outlet to a discharge conveyor 95 to be carried away from the chamber 91 to a succeeding step. A shower nozzle 96 is disposed at the outlet from the chamber 91 for injecting an aseptic water, thus flushing away any H₂O₂ gas injected from H₂O₂ gas injector 87 which may be attached to the vessels 4.

Each space within the aseptic chamber 82 and the chamber 91 in which the filler 89 is disposed are controlled to a positive pressure, and the pressures in these spaces are chosen as follows: specifically, the space 82B in which the second rotary wheel 84 is disposed exhibits a higher pressure than the space 82A in which the first rotary wheel 83 is disposed, and the interior of the electron beam irradiation chamber 85 exhibits a higher pressure than the space 82B in which the second rotary wheel 84 is disposed. In addition, the interior of the chamber 91 in which the filler 89 is disposed exhibits a slightly higher pressure than the interior of the electron beam irradiation chamber 85. The space 82C between the electron beam irradiation chamber 85 and the chamber 91 in which the filler 89 is disposed or the space 82C in which part of the discharge gripper wheel 18 and the intermediate rotary wheel 88 are disposed exhibits a pressure which is either higher or lower than both the upstream and the downstream space 85, 91.

The arrangement of the vessel inversion conveyer 10 will now be described with reference to Fig. 1 and Figs. 2 to 5. It is to be noted that Fig. 2 is a perspective view showing the arrangement of the vessel holder means 14, Fig. 3 is a cross section taken along the line III-III shown in Fig. 1, Fig. 4 is a cross section taken along the line IV-IV shown in Fig. 1, and Fig. 5 is a cross section taken along the line V-V shown in Fig. 1. A plurality of vessel holder means 14 are mounted around the outer periphery of the revolving body 12 (not entirely shown) at an equal circumferential interval. Referring to Fig. 2 for a detailed description of the arrangement of the vessel holder means 14, in this embodiment, a plurality of mounting blocks 50, which are channel-shaped in section, are horizontally secured to the outer periphery of the revolving body 12 at an equal circumferential interval with its opening 50a directed radially outward, and the vessel holder means 14 is mounted on each of the mounting blocks 50. Each vessel holder means 14 has a pair of vessel holders 26, 26. It is to be noted that the vessel holder means 14 of the present embodiment is constructed in a similar manner as the vessel holder means invented by the present inventors and disclosed in Japanese Laid-Open Patent Application No. 2003-192095 filed by the present applicant, but may be constructed otherwise.

In the present embodiment, the pair of vessel holders 26, 26 are constructed in an identical manner, and accordingly, corresponding parts are designated by like characters for purpose of description. Two parallel leaf springs (support plates) 56 having their centers secured to the opposite ends of a rod 54 are rotatably supported by the opening 50a of the mounting block 50. As mentioned previously, the mounting block 50 is secured to the outer periphery of the revolving body 12 and directed radially, and accordingly, the rod 54 which is supported across opening 50a is directed tangentially of the revolving body 12. Springs 58 are connected between the both leaf springs 56 on the opposite sides of the rod 54, thus normally urging the ends 56a of the both leaf springs 56 toward each other. A pair of holding plates 60 which oppose to each other are mounted to the opposite ends 56a (upper and lower ends as viewed in Fig. 2) of the two leaf springs 56, and the pair of opposing holding plates 60 constitute the vessel holder 26, 26. The opposing surfaces of the holding plates 60 are centrally formed with arcuate concave surfaces 60a which are adapted to abut against the neck of the vessel 4 (which is a portion 4c below a flange 4b in this embodiment), and also with outwardly enlarging guide surfaces 60b formed on the opposite sides thereof.

The holding plates 60 on the opposite sides which define the vessel holders 26, 26 are secured to the ends 56a of the pair of leaf springs 56, and are attracted toward each other by the springs 58. The portion 4c of the vessel 4 which is located below the flange 4b passes over the guide surfaces 60b of the opposing holding plates 60 to enter the arcuate concave surfaces 60a while forcing the holding plates 60 outward, and subsequently the springs 58 cause the opposing holding plates 60 to return, whereby the portion 4c of the vessel 4 is carried. Since each holding plate 60 is formed with guide surfaces 60b on the opposite sides of the arcuate concave surface 60a, the portion 4c can be seated between the opposing arcuate concave surfaces 60a or removed therefrom through the guide surfaces 60b on either side. The leaf springs 56, the springs 58 and the vessel holders 26, 26 constructed with one set of holding plates 60 constitute together the vessel holder means 14. The vessel holders 26, 26 of the vessel holder means 14 of the present embodiment are disposed at positions which are symmetrical to the center axis of the central rod 54 or the axis O1 which is tangential of the revolving body 12, allowing two vessels 4 to be carried in vertical alignment.

An engaging member 64 having opposite ends in which U-shaped recesses 64a engaging a guide rail 62 of the inversion means is secured to the center of the rod 54 as viewed in its lengthwise direction. As shown in Figs. 2-5, the engaging member 64 is mounted at an angle to be slanted relative to the opposing leaf springs 56. On the other hand, the guide rail 62 is disposed around the outer periphery of the revolving body 12 for engaging the U-shaped recess 64a in the engaging member 64 to rotate the vessel holder means 14 through about 180° about the axis O1 of the rod 54 as the revolving body 12 rotates so as to interchange the pair of upper and lower vessel holders 26, 26. As shown in Figs. 4 and 5, the guide rail 62 is supported by guide rail support means 63a which are mounted on a stationary stanchion 63 installed externally of the revolving body 12 so as to be positioned out of interference with the vessels 4 or the vessel holder means 14. At the vessel supply position A (see Figs. 1 and 4) where the vessels 4 are supplied from the supply gripper wheel 6 to the vessel inversion conveyor 10, the distal end 62a of the guide rail 62 (see Fig. 2) which is located toward the supply gripper wheel 6 (to the left as viewed in Fig. 4) becomes engaged with the U-shaped recess 64a.

When shifting toward the vessel discharge position B where the vessels 4 are discharged from the vessel inversion conveyor 10 to the discharge gripper wheel 18, the guide rail 62 gradually shifts inward of the revolving body 12 while increasing its elevation, followed by a descent. In other words, the position of the guide rail 62 rotates through 180° while twisting around the position passed by the rod 54. The vessel holder means 14 and the vessel 4 shown in broken lines in Fig. 4 indicate their conditions during the inversion, and when the position is changed through 180° as mentioned above, the two vessels 4 are completely interchanged up and down. In the present embodiment, the inversion begins at a position (inversion beginning position C) which is located slightly downstream of the vessel supply position A shown in Fig. 1. Specifically, the two vessel holders 26, 26 begin to become slanted from their vertically upright condition. At an intermediate position D, the both vessel holders 26, 26 assume a substantially horizontal condition, and the inversion is terminated at a position (inversion termination position E) which is located slightly upstream of the electron beam irradiator 24. In this manner, the pair of upper and lower vessel holders 26, 26 are interchanged and assume an upright condition. Consequently, the inversion interval extends from the inversion beginning position C to the inversion termination position E and a region extending from the inversion terminating position E through the vessel discharge position B and the vessel supplying position A to the inversion beginning position C represents an upright transfer interval where the two vessels 4, 4 which are carried in vertical alignment by the two vessel holders 26, 26 are transferred while their axes are directed in the vertical direction. The electron beam irradiator 24 is disposed in the upright transfer interval extending from the inversion termination position E to the vessel discharge position B. The position of the vessel inversion conveyor 10 where the irradiation unit 24a of the electron beam irradiator 24 is directed represents the electron beam irradiation position F.

The operation of the vessel sterilizing and filling line constructed in the manner mentioned above will now be described. The vessels 4 which are conveyed while being suspended from the air conveyor 80 are separated to a given interval by the in-feed screw 81 to be introduced into the aseptic chamber 82 which is made from lead where they are handed to the first rotary wheel 83, and then handed to the second rotary wheel 84 which is partitioned therefrom by the partition wall 82a. H₂O₂ gas injectors 87 are mounted at two locations within the space 82B in which the second rotary wheel 84 is disposed, and the injection nozzle 87a injects a gas which is obtained by gasifying H₂O₂ solution. The inactive gas injection nozzle 97 is installed in the region G of the second rotary wheel 84, and an inactive gas such as nitrogen gas is blown from the injection nozzle 97 into the vessels 4 being conveyed, thus reducing the amount of oxygen within the vessels 4 as much as possible. Subsequently, the vessels 4 are fed through the second rotary wheel 84 to be handed to the gripper 8 of the supply gripper wheel 6 to be rotatively conveyed while being carried by the gripper 8. In the present embodiment, the gripper 8 of the supply gripper wheel 6 carries a portion 4d of the vessel 4 which is located above the flange 4b formed around the neck (see Fig. 4).

The second rotary wheel 84 and the supply gripper wheel 6 are partitioned from each other by the partition wall 82b except for a region where the vessels are handed over between them 84, 86, and the vessel 4 is handed from the second rotary wheel 84 to the gripper 8 of the supply gripper wheel 6 to be conveyed into the electron beam irradiation chamber 85. H₂O₂ gas injector 87 is mounted at three locations within the electron beam irradiation chamber 85 for injecting a gas which is gasified from H₂O₂ solution by the injection nozzle 87a. For an electron beam sterilizer, the germicidal power is ineffective except for a region which is irradiated directly with the electron beam, but in the present embodiment, the entire electron beam irradiation chamber 85 maintains a germicidal H₂O₂ gas atmosphere.

As the vessel 4 carried by the gripper 8 of the supply gripper wheel 6 approaches the supply position A with respect to the vessel inversion conveyor 10, the portion 4c which is located below the flange 4b is inserted between the opposing holding plates 60 of the lower one of the upper and lower vessel holders 26, 26 (see the lower vessel holder 26 shown in Fig. 4 which indicates the vessel supply position A) of the vessel holder means 14 which is mounted on the channel-shaped mounting member 50 secured at an equal circumferential interval around the revolving body 12 of the vessel inversion conveyor 10. As shown in solid line in Fig. 4, the two vessel holders 26, 26 are in vertical alignment at the supply position A from the supply gripper wheel 6 to the vessel inversion conveyor 10.

The both holding plates 60 are secured to the leaf springs 56 and are attracted toward each other by means of the springs 58, and accordingly, the vessel 4 which is carried by the gripper 8 of the supply gripper wheel 6 passes over the guide surfaces 60b of the both holding plates 60 while forcing them apart to be snapped into the arcuate recesses 60a. At the commencement of the operation of the vessel inversion conveyor 10, only the lower holder 26 shown in Fig. 4 carries the vessel 4. On the other hand, the engaging member 64 which is integral with the vessel holder means 14 has its downwardly directed U-shaped recess 64a engaged with the distal end of the 62a of the guide rail 62. At this point in time, the upwardly directed U-shaped recess 64a is engaged by the distal end 62b of the guide rail 62 (see Fig. 4).

As the supply gripper wheel 6 and the revolving body 12 of the vessel inversion conveyor 10 both rotate, and the gripper 8 and the vessel holder 26 of the vessel holder means 14 move apart, the vessel 4 is disengaged from the gripper 8 of the supply gripper wheel 6 and is carried by the vessel holder 26 to be rotatively conveyed as the revolving body 12 of the vessel inversion conveyor 10 rotates. An interval extending from the vessel supply position A to the inversion beginning position C which is located downstream represents the upright transfer interval. Upon passing through the inversion beginning position and entering the inversion interval (C-E), the U-shaped recess 64a of the engaging member 64 moves upwardly and radially inward in conformity to the configuration of the guide rail 62, thus causing the vessel holder means 14 to be rotated so as to interchange the upper and the lower vessel holder 26, 26. When the two vessel holders 26, 26 rotate through 180° and are interchanged between the upper and the lower one, the vessel 4 which is carried by one of the vessel holders 26 (which is the lower vessel holder shown in Fig. 4) is inverted from its erect condition at the lower position to the inverted condition at the upper position.

As the vessel holder means 14 passes through the inversion interval (C-E), the upper and the lower holder 26, 26 rotate through 180° about the rod 54, whereby the vessel 4 which is carried in its erect condition by the lower holder 26 assumes a completely inverted condition (see the upper vessel 4 shown in Fig. 5). A region extending from the inversion termination position E to the vessel discharge position B again represents the upright transfer interval, and the electron beam irradiator 24 is installed in this interval E-B. The irradiation unit 24a of the electron beam irradiator 24 is disposed so as to be directed radially inward of the revolving body 12 which constitutes the vessel inversion conveyor 10 (see the electron beam irradiation position F shown in Figs. 1 and 3), and irradiates the electron beam across the upper and the lower end of the passing vessel 4. During this irradiation (the first irradiation with respect to the vessel 4), substantially one-half surface which faces outward of the vessel 4 carried by the upper holder 26 of the vessel holder means 14 is sterilized with the electron beam (a hatched portion of the vessel 4 which is carried by the upper holder 26 as viewed in Fig. 3 is sterilized).

Subsequently, the revolving body 12 of the vessel inversion conveyor 10 continues to rotate, and the vessel holder 26 reaches the vessel discharge position B. The discharge gripper wheel 18 which is provided with the gripper 20 is disposed at the vessel discharge position B, and the gripper 20 is located at an elevation which allows it to grip the vessel 4 which is carried by the lower holder 26. At this point in time which immediately follows the commencement of the operation, the lower vessel holder 26 does not carry the vessel 4, and accordingly, the vessel holder means 14 passes the vessel discharge position B without effect.

When the vessel holder means 14 again reaches the supply position A, the gripper 8 of the supply gripper wheel 6 which receives the vessel 4 supplied from the second rotary wheel 84 to convey it rotatively hands the succeeding vessel 4 to the empty, lower vessel holder 26 of the vessel holder means 14. At this point in time, the upper and the lower vessel holder 26, 26 of the vessel holder means 14 each carry the vessels 4, 4. At this point, the two vessels 4, 4 are in vertical alignment as shown in Fig. 4, with the vessel carried by the lower holder 26 assuming its erect condition and the vessel 4 carried by the upper holder 26 assuming its inverted condition.

The vessel holder means 14 carrying the pair of upper and lower vessels 4, 4 in vertical alignment passes through the upright transfer interval A-C which extends to the inversion beginning position C, and again enters the inversion interval C-E, whereby it is inverted in accordance with the locus of movement of the guide rail 62, the upper vessel holder 26 moving to its lower position while the lower vessel holder 26 moving to its upper position. When it passes through the inversion termination position E and enters the upright transfer interval, it reaches the electron beam irradiation position F where the irradiation unit 24a of the electron beam irradiator 24 is installed to be irradiated with the electron beam. The vessel 4 which has been subject to the irradiation of the electron beam from the irradiation unit 24a during the previous run is inverted about the rod 54 which is directed tangentially of the revolving body 12 in the inversion interval C-E, and the portion which was irradiated with the electron beam during the previous run is located to the right as viewed in Fig. 3. Accordingly, the vessel 4 (the lower vessel 4 as viewed in Fig. 3) which passes the electron beam irradiation position F for the second time has its portion which has not been subjected to the irradiation of the electron beam during the previous run (or the portion located to the left as viewed in Fig. 3) is directed toward the electron beam irradiator 24, and the remaining portion of this vessel 4 and one-half of the vessel 4 carried by the upper vessel holder 26 which is located toward the electron beam irradiator 24 are subject to the irradiation of the electron beam across the upper and the lower end of these two vessels 4 to be sterilized. The vessel 4 which has been carried by the lower vessel holder 26 is now subject to the irradiation of the electron beam twice, thus when it assumes the upper position and when it assumes the lower position after the inversion, whereby the entire outer peripheral surface is sterilized.

In the present embodiment, H₂O₂ gas which is gasified from H₂O₂ solution is injected from the nozzle 87a of the H₂O₂ gas injector 87 into the electron beam irradiation chamber 85 which is used to irradiate the electron beam upon the conveyed vessel 4 for purpose of the sterilization, and H₂O₂ gas atmosphere is normally maintained therein. Accordingly, if bacilli is introduced from the outside of the aseptic chamber 82 by attachment to the vessel 4, for example, there is no likelihood that they will be re-attached to the sterilized vessel 4, thus providing an electron beam vessel sterilizer having a high sterilization reliability.

When the vessel holder means 14 reaches the vessel discharge position B after passing through the electron beam irradiation position F, the vessel 4 carried by the lower holder 26 is engaged by the gripper 20 of the discharge gripper wheel 18 to be taken out of the vessel holder 26 and rotatively conveyed to be discharged from the electron beam irradiation chamber 85 to be handed to the succeeding intermediate rotary wheel 88. On the other hand, the vessel holder means 14 from which the vessel 4 are taken out from the lower vessel holder 26 at the vessel discharge position B moves from the vessel discharge position B to the vessel supply position A while only the upper vessel holder 26 carries the vessel 4. At the vessel supply position A, the gripper 8 of the supply gripper wheel 6 hands the vessel 4 to the lower vessel holder 26. In this manner, one vessel 4 is conveyed to pass the inversion interval C-E and the electron beam irradiation position F twice by the rotation of the revolving body 12 of the electron beam sterilizer 10, and is subject to the irradiation of the electron beam twice, when it assumes the inverted condition by being carried by the upper vessel holder 26 of the vessel holder means 14 and when it assumes the erect condition by being carried by the lower vessel holder 26 as it passes in front of the irradiation unit 24a of the electron beam irradiator 24. Accordingly, the provision of the single electron beam irradiator 24 is sufficient to sterilize the entire outer peripheral surface completely during the time the vessel 4 is continuously conveyed.

The aseptic air is blown from the injection nozzle 98 into the sterilized vessel 4 which is handed from the discharge gripper wheel 18 to the intermediate wheel 88 during the time the vessel is conveyed through the region H where the aseptic air injection nozzle 98 is installed. The aseptic air is effective to blow the ozone off if the ozone generated by the irradiation with the electron beam happened to be attached to the vessel 4. Subsequently, the vessel passes through the partition wall 82d to enter the separate chamber 91 in which the filler 89 and the capper 90 are disposed, and is handed from the intermediate rotary wheel 88 to the supply wheel 92 for the filler 89, whereby it is supplied to the filler 89. The vessel 4 supplied into the filler 89 is rotatively conveyed while it is filled with a liquid, and then discharged from the filler 89 through the discharge wheel 93 to be fed to the capper 90. A capping operation takes place in the capper 90, and then the vessel is handed to the discharge conveyor 95 through the delivery wheel 94 which is disposed at the outlet and conveyed by the discharge conveyor 95 to be discharged from the chamber 91. The aseptic water shower nozzle 96 is disposed at the outlet from the chamber 91, and an aseptic water is injected to the capped vessel 4. During the sterilization in the electron beam irradiation chamber 85, H₂O₂ gas which is injected into the electron beam irradiation chamber 85 may be attached to the vessel, but may be flushed off by the injection of the aseptic water.

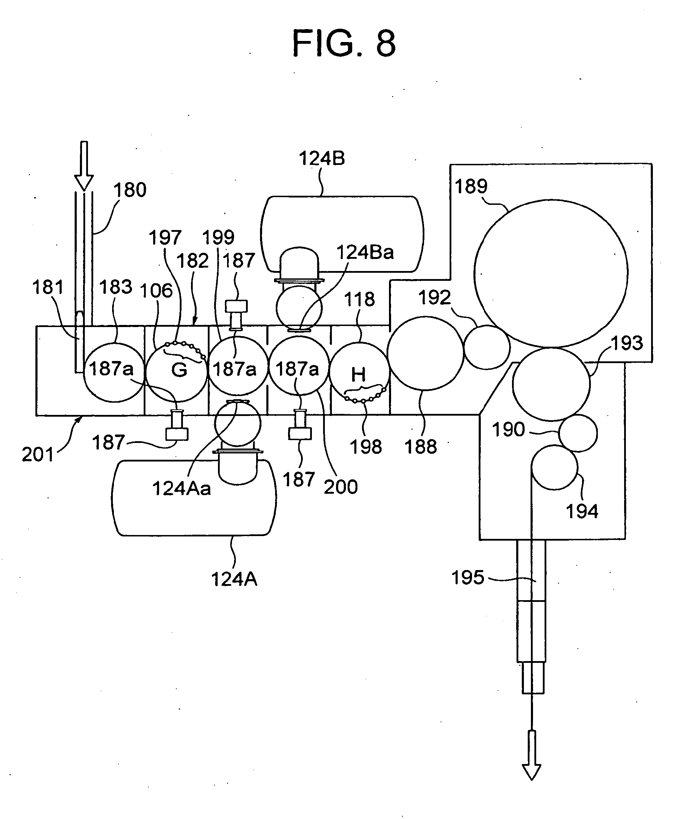

Fig. 8 is a plan view showing an entire arrangement of a vessel sterilizing and filling line which is provided with a vessel sterilizer according to a second embodiment. In the first embodiment, the vessel inversion conveyor 10 inverted the vessels 4 while conveying them, and the single electron beam irradiator 24 performed the irradiation of the electron beam twice to achieve the sterilization of the entire surface of the vessel 4. However, in this embodiment, a neck conveyance is made in which the neck 4c of the vessel 4 is supported to suspend it during the conveyance. Because the vessel 4 is not inverted, the irradiation of the electron beam in one direction cannot sterilize the entire outer surface of the vessel 4, and accordingly, the irradiation of the electron beam takes place twice by two electron beam irradiators 124A, 124B which are directed oppositely through 180°.

Vessels which are conveyed by an air conveyor 180 are separated to a given interval by an in-feed screw 181 to be introduced into a chamber 201 where they are handed to a rotary wheel 183. Subsequently, then the vessels are fed successively through a supply wheel 106 to first rotative conveying means 199 and second rotative conveying means 200 which constitute together a vessel sterilizer according to this embodiment. Then the vessels are fed through a discharge wheel 118 and an intermediate wheel 188 to a supply wheel 192, which supplies the vessels to a filler 189. The supplied vessels 4 are filled with a liquid in the filler 189, and taken out of the filler 189 by an intermediate wheel 193 to be introduced into the capper 190. The vessels 4 which are capped by the capper 190 is fed through a delivery wheel 194, disposed at the outlet, to a discharge conveyor 195 to be fed to a succeeding step.

Components located from the rotary wheel 183 which receives the vessels 4 supplied from the air conveyor 180 through the in-feed screw 181 to the delivery wheel 194 disposed at the outlet and which delivers the vessels 4 which are capped by the capper 199 to the discharge conveyor 195 are contained within a chamber 201. The first rotative conveying means 199 and the second rotative conveying means 200 which constitute together the vessel sterilizer according to this embodiment as well as the supply wheel 106 which is disposed upstream thereof are contained in an aseptic chamber 182 in the similar manner as the lead chamber 82 of the first embodiment. A first electron beam irradiator 124A which opposes the first conveying means 199 is mounted on one of sidewalls of the lead aseptic chamber 182 (or lower sidewall as viewed in Fig. 6) while a second electron beam irradiator 124B which opposes the second conveying means 200 is mounted on the other sidewall (or the lower sidewall as viewed in Fig. 6). These electron beam irradiators 124A, 124B include irradiation units 124Aa, 124Ba, which are directed oppositely by 180°. H₂O₂ gas injectors 187 are mounted on the wall surface which is located opposite to the first electron beam irradiator 124A, on the wall surface which is located opposite to the second electron beam irradiator 124B, and on the wall surface of the space in which the supply gripper wheel 106 is disposed. These H₂O₂ gas injectors 187 each include a nozzle 187a which injects H₂O₂ gas to form an atmosphere within the lead, aseptic chamber 182.

In the similar manner as in the first embodiment, an inactive gas injection nozzle 197 is disposed within a conveying region G of the supply wheel 106, and an aseptic air injection nozzle 198 is disposed within a conveying region H of the discharge wheel 118 so as to inject an inactive gas such as nitrogen gas and an aseptic air into the vessels 4.

In this embodiment, one-half the outer surface of the vessel 4 which is subject to a neck conveyance by the first rotative conveying means 199 and which faces the first electron beam irradiator 124A is sterilized by the irradiation of the electron beam, followed by the irradiation of the electron beam upon one-half outer surface of the vessel 4 which is handed over to the second rotative conveying means 200 to be subject to a neck conveyance and which faces the second electron beam irradiator 124b, whereby the entire outer peripheral surface of the vessel is sterilized. Since H₂O₂ gas atmosphere is maintained within the aseptic chamber 182 which is made from lead and in which the sterilization by way of the electron beam takes place. The likelihood that germs attached to the vessels which are conveyed into the chamber from the exterior and floating within the chamber may be re-attached to the sterilized vessels 4 is avoided inasmuch as these germs are sterilized by H₂O₂ gas.

## Claims

1. A vessel sterilizer including vessel conveying means (10) disposed within a chamber (82) and an electron beam irradiator (24) which is arranged to irradiate vessels (4) conveyed by the vessel conveying means (10) with an electron beam;
**characterized in that** a nozzle (89) is disposed within the chamber for injecting a germicidal gas;
the nozzle (89) is configured to inject germicidal gas into the chamber (82) to allow the condition that a germicidal gas atmosphere is maintained in the chamber (82); and
the sterilizer is configured to irradiate the vessels (4) with the electron beam while the germicidal gas atmosphere is maintained in the chamber (82).

2. A vessel sterilizer according to Claim 1, **characterized by** an inactive gas injector (97) disposed at a location upstream of the vessel conveying means (10) for injecting an inactive gas into the vessel (4).

3. A vessel sterilizer according to Claim 1 or 2, **characterized by** a filler (89) disposed downstream of the vessel conveying means (10) for filling the vessel (4) with a liquid, and air injection means (98) disposed between the vessel conveying means (10) and the filler (89) for injecting aseptic air into the vessel.

4. A vessel sterilizer according to Claim 1, 2 or 3 in which the germicidal gas is formed by gasifying hydrogen peroxide solution.

5. A method of sterilizing vessels (4) which are conveyed within a chamber (82) by irradiating the vessels (4) with an electron beam,
**characterized by** the steps of:
injecting a germicidal gas into the chamber to maintain a germicidal gas atmosphere in the chamber (82); and
irradiating the vessels (4) with the electron beam while the germicidal gas atmosphere is maintained in the chamber (82).

6. A method of sterilizing vessels (4) according to Claim 5 **characterized in that** an inactive gas is injected into the vessels (4) to reduce an amount of oxygen within the vessels before the irradiation of the vessels (4) with the electron beam.

7. A method of sterilizing vessels (4) according to Claim 5 or 6 **characterized in that** aseptic air is injected into the vessels (4) before the sterilized vessels (4) are filled with a filling liquid.

8. A method of sterilizing vessels (4) according to Claim 5, 6 or 7 **characterized in that** the germicidal gas is formed by gasifying hydrogen peroxide solution.

## Patentansprüche

1. Gefäßsterilisator, der ein in einer Kammer (82) angeordnetes Gefäßfördermittel (10) aufweist und eine Elektronenbestrahlungseinrichtung (24), die angeordnet ist, um Gefäße (4) mit einem Elektronenstrahl zu bestrahlen, die durch das Gefäßfördermittel (10) befördert werden;
**dadurch gekennzeichnet, dass** eine Düse (89) in der Kammer zum Einspritzen eines keimtötenden Gases angeordnet ist; wobei
die Düse (89) eingerichtet ist, keimtötendes Gas in die Kammer (82) einzuspritzen, um den Zustand zu ermöglichen, dass in der Kammer (82) eine keimtötende Gasatmosphäre aufrechterhalten wird; und
der Sterilisator eingerichtet ist, die Gefäße (4) mit dem Elektronenstrahl zu bestrahlen während die keimtötende Gasatmosphäre in der Kammer (82) aufrechterhalten wird.

2. Gefäßsterilisator nach Anspruch 1, **gekennzeichnet durch** eine inaktive Gaseinspritzeinrichtung (97), die an einer vorgelagerten Stelle zu dem Gefäßfördermittel (10) zum Einspritzen eines inaktiven Gases in das Gefäß (4) angeordnet ist.

3. Gefäßsterilisator nach Anspruch 1 oder 2, **gekennzeichnet durch** eine Fülleinrichtung (89), die dem Gefäßfördermittel (10) zum Füllen des Gefäßes (4) mit einer Flüssigkeit nachgelagert angeordnet ist, und ein Lufteinspritzmittel (98), das zwischen dem Gefäßfördermittel (10) und der Fülleinrichtung (89) zum Einspritzen aseptischer Luft in das Gefäß angeordnet ist.

4. Gefäßsterilisator nach Anspruch 1, 2 oder 3, in dem das keimtötende Gas durch Vergasen einer Wasserstoffperoxidlösung gebildet wird.

5. Verfahren zum Sterilisieren von Gefäßen (4) durch Bestrahlen der Gefäße (4) mit einem Elektronenstrahl, die in einer Kammer (82) befördert werden,
**gekennzeichnet durch** die Schritte:
Einspritzen eines keimtötenden Gases in die Kammer, um eine keimtötende Gasatmosphäre in der Kammer (82) aufrechtzuerhalten; und
Bestrahlen der Gefäße (4) mit einem Elektronenstrahl, während die keimtötende Gasatmosphäre in der Kammer (82) aufrechterhalten wird.

6. Verfahren zum Sterilisieren von Gefäßen (4) nach Anspruch 5, **dadurch gekennzeichnet, dass** ein inaktives Gas in die Gefäße (4) eingespritzt wird, um vor dem Bestrahlen der Gefäße (4) mit dem Elektronenstrahl eine Menge an Sauerstoff in den Gefäßen zu reduzieren.

7. Verfahren zum Sterilisieren von Gefäßen (4) nach Anspruch 5 oder 6, **dadurch gekennzeichnet, dass** aseptische Luft in die Gefäße (4) eingespritzt wird, bevor die sterilisierten Gefäße (4) mit einer Flüssigkeit gefüllt werden.

8. Verfahren zum Sterilisieren von Gefäßen (4) nach Anspruch 5, 6 oder 7, **dadurch gekennzeichnet, dass** das keimtötende Gas durch Vergasen einer Wasserstoffperoxidlösung gebildet wird.

## Revendications

1. Stérilisateur de récipients incluant un moyen de transport de récipients (10) disposé à l'intérieur d'une chambre (82) et un faisceau d'électrons irradiant (24) qui est agencé de manière à irradier des récipients (4) transportés par le moyen de transport de récipients (10) avec un faisceau d'électrons ;
**caractérisé en ce qu'**une buse (89) est disposée à l'intérieur de la chambre pour injecter un gaz germicide ;
la buse (89) est configurée pour injecter le gaz germicide à l'intérieur de la chambre (82) de façon à permettre une condition où une atmosphère de gaz germicide est maintenue dans la chambre (82) ; et
le stérilisateur est configuré pour irradier les récipients (4) avec le faisceau d'électrons tandis que l'atmosphère de gaz germicide est maintenue dans la chambre (82).

2. Stérilisateur de récipients selon la revendication 1, **caractérisé par** un injecteur de gaz inactif (97) disposé en un emplacement en amont du moyen de transport de récipients (10) pour injecter un gaz inactif dans le récipient (4).

3. Stérilisateur de récipients selon la revendication 1 ou 2, **caractérisé par** un dispositif de remplissage (89) disposé en aval du moyen de transport de récipients (10) pour remplir le récipient (4) avec un liquide, et un moyen d'injection d'air (98) disposé entre le moyen de transport de récipients (10) et le dispositif de remplissage (89) pour injecter de l'air aseptique à l'intérieur du récipient.

4. Stérilisateur de récipients selon la revendication 1, 2 ou 3, dans lequel le gaz germicide est formé par gazéification d'une solution de peroxyde d'hydrogène.

5. Procédé de stérilisation de récipients (4) qui sont transportés à l'intérieur d'une chambre (82) par irradiation des récipients (4) avec un faisceau d'électrons,
**caractérisé par** les étapes :
d'injection d'un gaz germicide à l'intérieur de la chambre pour maintenir une atmosphère de gaz germicide dans la chambre (82) ; et
d'irradiation des récipients (4) avec le faisceau d'électrons tandis que l'atmosphère de gaz germicide est maintenue dans la chambre (82).

6. Procédé de stérilisation de récipients (4) selon la revendication 5 **caractérisé en ce qu'**un gaz inactif est injecté à l'intérieur des récipients (4) pour réduire une quantité d'oxygène à l'intérieur des récipients avant l'irradiation des récipients (4) avec le faisceau d'électrons.

7. Procédé de stérilisation de récipients (4) selon la revendication 5 ou 6 **caractérisé en ce qu'**un gaz aseptique est injecté à l'intérieur des récipients (4) avant que les récipients (4) stérilisés ne soient remplis avec un liquide de remplissage.

8. Procédé de stérilisation de récipients (4) selon la revendication 5, 6 ou 7 **caractérisé en ce que** le gaz germicide est formé par gazéification d'une solution de peroxyde d'hydrogène.
